# EUROPÄISCHE PATENTSCHRIFT

(11) **EP 1 857 068 B1**
(45) Veröffentlichungstag und Bekanntmachung des Hinweises auf die Patenterteilung: **25.02.2009**
(21) Anmeldenummer: 06009745.8
(22) Anmeldetag: 11.05.2006
(51) Int. Cl.: A61B 19/00, A61B 17/00

(54) **Vorrichtung zum Tracken von starren, schwer oder unmöglich zu registrierenden Körperstrukturen**
Device for tracking rigid, difficult or impossible to register body structures
Dispositif de suivi pour des structures corporelles difficiles à enregistrer

(43) Veröffentlichungstag der Anmeldung: 21.11.2007
(73) Patentinhaber: BrainLAB AG, 85622 Feldkirchen (DE)
(72) Erfinder: Wörlein, Swen, 80637 München (DE); Witte, Jens, 80339 München (DE); Frielinghaus, Nils, 85551 Heimstetten (DE)
(74) Vertreter: Schwabe - Sandmair - Marx

(56) Entgegenhaltungen:
- WO-A-99/21498
- DE-A1- 4 304 570
- DE-A1- 10 135 156
- US-B1- 6 205 411

## Beschreibung

Die vorliegende Erfindung betrifft eine Vorrichtung zum Tracken oder Verfolgen mindestens eines starren Objektteils, wie eines starren Körper- oder Knochenstrukturteils eines Patienten, welches von einem starren Referenzobjekt, wie einer starren Körper- oder Knochenstruktur des Patienten, ablösbar oder abnehmbar ist, mittels dessen das starre Objektteil ohne Erfassung von Referenzpunkten auf dem Objektteil von einem bildgestützten Navigationssystem oder einem Image-Guided-Surgery-System (IGS-System) getrackt oder verfolgt werden kann, so dass die Positionen des Objektteils in einem Patientendatensatz, wie CT-Aufnahmen, dargestellt werden können. Es wird auch ein Verfahren zum Versetzen oder Neupositionieren oder Austauschen des Objektteils in dem Referenzobjekt an eine vorgegebene oder vorbestimmte Position beschrieben, wobei durch Tracken oder Bestimmen der Position des Objektteils das Objektteil an die vorgegebene oder vorbestimmte Position gesetzt werden kann.

In herkömmlichen Verfahren werden Knochenstrukturen registriert, um das Tracken dieser Knochenstrukturen zu ermöglichen.

Die US 6,236,875 betrifft Systeme, welche Bilder, die zwischen medizinischen und chirurgischen Eingriffen aufgenommen werden, erzeugen und verwenden, wobei die Bilder bei der Durchführung der Eingriffe hilfreich sind und die relative Position von verschiedenen Körperteilen und Instrumenten darstellen.

Bei herkömmlichen Verfahren müssen Punkte auf den zu trackenden oder verfolgenden Körperteilen erfasst werden und deren Positionen ermittelt werden, um eine Registrierung des Körperteils und ein Tracken des Körperteils zu ermöglichen.

Aus der WO 99/21498 ist ein System zur Knochensegmentnavigation bekannt, wobei eine anhand eines präoperativen Datensatzes angefertigte Schablone zusammen mit einer Markeranordnung in eindeutiger Lage- und Orientierungsbeziehung auf einem Knochensegment angeordnet wird. Die Position dieser Markeranordnung kann mit einer Positionserfassungseinheit erfasst werden, so dass die Anfangsposition des Knochensegments berechnet und als Konturlinienzug dargestellt werden kann.

Die US 6,205,411 B1 offenbart eine Vorrichtung zur Ermöglichung der Implantierung einer künstlichen Gelenkkomponente und umfasst einen prä-operativen geometrischen Planer und einen prä-operativen kinematischen biomechanischen Simulator in Verbindung mit dem präoperativen geometrischen Planer. Während der intra-operativen Schritte des Verfahrens wird ein Computersystem verwendet, um die relativen Orte der mit einer Tracking-Vorrichtung getrackten Objekte anzuzeigen. Das Gelenk-Modell basierend auf den Skelett-Daten wird bezüglich der intra-operativen Position des Gelenks des Patienten ausgerichtet Eine dreidimensionale Oberflächenregistrierung wird durchgerührt, wobei diskrete Registrier-Punkte von der Gelenk-Skelett-Struktur erhalten werden, um die intra-operative Position des Gelenks des Patienten zu bestimmen. Die Registrier-Punkte werden an das Gelenk-Modell der Skelett-Struktur angepasst, um eine Koordinatentransformation zu bestimmen, welche verwendet wird, um das Gelenk-Modell bezüglich der intra-operativen Position des Gelenks des Patienten auszurichten. Wird beispielsweise eine femorale Osteotomie geplant, so werden die Knochenschnittebenen prä-operativ positioniert und interaktiv gedreht. Mittels eines präoperativen kinematischen biomechanischen Simulators wird der Bewegungsbereich (range of motion) für ausgewählte Testbewegungen berechnet. Die Knochenfragmente und Instrumente werden getrackt und die Knochensegmente werden intra-operativ neu positioniert.

Es ist eine Aufgabe der vorliegenden Erfindung, die bestehenden Nachteile aus dem Stand der Technik zu überwinden. Es ist weiter eine Aufgabe der vorliegenden Erfindung eine Vorrichtung bereitzustellen, welche ein schnelles und einfaches Tracken oder Neupositionieren kleiner, schlecht oder unmöglich zu registrierender starrer Objektteile oder Knochenstrukturteile ermöglichen.

Diese Aufgabe wird durch den Gegenstand des unabhängigen Anspruchs gelöst. Vorteilhafte Ausführungsformen ergeben sich aus den abhängigen Ansprüchen.

Im Sinne der vorliegenden Erfindung wird als ein starres Objekt eine Körperstruktur und als ein starres Objektteil ein Teil einer Körperstruktur bezeichnet, welche ihre Form zwischen der Aufnahme der Körperstruktur oder des Körperstrukturteils und einem medizinischen Vorgang oder Eingriff nicht ändern und nicht zusammengedrückt oder zusammengepresst oder verformt werden.

Bei einem Verfahren zum Tracken oder Verfolgen mindestens eines starren Objektteils, insbesondere eines starren Körper- oder Knochenstrukturteils eines Patienten, wird das Objektteil, welches von einem starren Referenzobjekt, insbesondere einer starren Körper- oder Knochenstruktur des Patienten, ablösbar oder abnehmbar ist, mittels eines bildgestützten Navigationssystems oder eines Image-Guided-Surgery-Systems (IGS-System) getrackt oder verfolgt.

Dabei wird ein Patientendatensatz, wie ein Computertomographie-Datensatz (CT-Datensatz) oder ein anderer medizintechnischer Datensatz des Patienten, in welchem starren Körper- oder Knochenstrukturen eines Patienten darstellbar sind, bereitgestellt oder ermittelt. Beispielsweise kann ein vor der Durchführung des Verfahrens aufgenommener Patientendatensatz, wie zum Beispiel ein mittels eines Computertomographen ermittelter Patientendatensatz, verwendet werden oder es kann vor oder während oder zwischen Einzelschritten des Verfahrens zum Tracken des starren Objektteils der Patientendatensatz, beispielsweise mittels eines Computertomographen oder eines anderen medizinischen bildgebenden Systems, ermittelt werden. Der aufgenommene oder bereitgestellte Patientendatensatz kann ein Datensatz, insbesondere mindestens eine Aufnahme, einer Region um das Objektteil oder des Körper- oder Knochenstrukturteils oder zumindest eines Teils des Objekts oder Referenzobjekts oder der starren Körper- oder Knochenstruktur sein.

In dem aufgenommenen oder bereitgestellten Patientendatensatz wird das Objektteil oder das Knochenstrukturteil, welches mit dem Objekt oder Referenzobjekt beispielsweise ablösbar oder abnehmbar verbunden ist oder welches an dem Objekt oder Referenzobjekt beispielsweise ablösbar oder abnehmbar angeordnet ist, markiert. Insbesondere kann das Objektteil durch Anzeichnen oder Auswählen in einer Darstellung des Patientendatensatzes von einem Arzt markiert werden und zum Beispiel in dem Patientendatensatz hervorgehoben oder eingefärbt werden. Auch kann das markierte Objektteil oder können die markierten Objektteile vorzugsweise halbautomatisch oder automatisch markiert und segmentiert werden. Insbesondere kann auch das Objektteil in dem Patientendatensatz durch Platzierung von Resektionslinien markiert werden. Vorzugsweise wird durch Markierung des Objektteils das Objektteil in dem Patientendatensatz, wie den Patientenaufnahmen, farbig hervorgehoben oder in dem Patientendatensatz segmentiert, so dass es in einer Darstellung des Patientendatensatzes relativ zu dem übrigen Patientendatensatz oder zu dem Referenzobjekt bewegt oder verschoben werden kann.

Vorzugsweise wird als Referenzobjekt eine Schädelknochenstruktur, insbesondere der Schädelknochen, des Patienten und als Objektteil ein Knochen der Schädelknochenstruktur oder des Schädelknochens, wie das Stirnbein, das Jochbein; der Oberkiefer, das Zwischenkieferbein, der Unterkiefer, das Nasenbein, das Tränenbein, das Gaumenbein, das Pflugscharbein oder das Zungenbein verwendet, um beispielsweise das jeweilige Knochenstrukturteil bei einem oralchirurgischen Eingriff zu tracken oder dessen Position zu ermitteln und darzustellen. Auch kann als Referenzobjekt eine Armknochenstruktur oder eine Beinknochenstruktur verwendet werden und ein davon ablösbares oder abnehmbares Knochenstrukturteil als Objektteil verwendet werden.

Auf dem Referenzobjekt oder an dem Referenzobjekt wird ein erster Referenzstern an einer beliebigen oder vorgegebenen Position angeordnet oder angebracht. Die Position des ersten Referenzsterns befindet sich auf dem Referenzobjekt oder auf einem mit dem Referenzobjekt verbundenen Teil oder Objektteil in einem festen Abstandsverhältnis zu dem mindestens einen markierten oder zu trackenden oder zu verfolgenden Objektteil und befindet sich nicht auf dem markierten oder zu trackenden oder zu verfolgenden Objektteil. Die Position des ersten Referenzsterns kann mittels des bildgestützten Navigationssystems erfasst und ermittelt werden oder kann von dem Navigationssystem vorgegeben werden oder dem Navigationssystem bekannt sein.

Mittels mechanischer Berührungen oder berührungslos, wie zum Beispiel mittels eines Laser-Pointers, werden Referenzpunkte auf dem Referenzobjekt erfasst und zum Beispiel dem Navigationssystem drahtlos oder drahtgebunden übermittelt, so dass unter Berücksichtung der von dem Navigationssystem erfassten Position oder dem Navigationssystem bekannten Position des ersten Referenzsterns den erfassten Referenzpunkten dreidimensionale Raumpositionen bezüglich eines Referenzkoordinatensystems oder Weltkoordinatensystems, wie dem ersten Refernzstern, zugeordnet werden können. Es kann eine Vielzahl von Referenzpunkten erfasst werden, um das Referenzobjekt bezüglich des ersten Referenzsterns oder bezüglich des Referenzkoordinatensystems zu registrieren.

Mittels des IGS-Systems oder des Navigationssystems oder Trackingsystems werden die ermittelten dreidimensionalen Raumpositionen der Referenzpunkte des Referenzobjekts den korrespondierenden Punkten oder Stellen oder Positionen in dem Patientendatensatz zugeordnet. Damit kann ein Teil des Objekts oder kann das Objekt bezüglich des ermittelten oder bereitgestellten Patientendatensatzes registriert werden, wobei insbesondere eine Region um das zu verfolgende Objektteil registriert wird. Damit sind die Punkte auf dem Objekt oder der Knochenstruktur oder ist die Oberfläche des Objekts oder der Knochenstruktur zumindest nahezu eindeutig dem Patientendatensatz zuordenbar, so dass Veränderungen an dem Objekt oder Verschiebungen an dem Objekt oder Verschiebungen von Objektteilen relativ zu dem Objekt in dem Patientendatensatz, wie den Aufnahmen des Objekts, darstellbar sind.

Auf dem zu verfolgenden oder zu trackenden Objektteil wird vor dem Ablösen oder Abnehmen des Objektteils von dem Objekt ein zweiter Referenzstern angeordnet, wobei der Abstand oder das Abstandsverhältnis zwischen dem zweiten Referenzstern und dem ersten Referenzstern vorgegeben sein kann oder vorzugsweise von dem Navigationssystem oder dem IGS-System ermittelt werden kann. Damit ist dem Navigationssystem oder dem IGS-System der Abstand des auf dem Objektteil angeordneten zweiten Referenzsterns von dem auf dem Objekt angeordneten ersten Referenzstern bekannt, so dass dem Navigationssystem die Position des zweiten Referenzsterns im Raum bezüglich des Referenz- oder Weltkoordinatensystems, wie des ersten Referenzsterns, bekannt ist.

Auch kann die Form und/oder Größe des Objektteils oder Knochenstrukturteils dem Navigationssystem, zum Beispiel durch die Definition von Resektionslinien, bekannt sein oder von dem Navigationssystem festgestellt werden. Auch kann das Objektteil oder Knochenstrukturteil vor dem Ablösen des Objektteils einen festen Abstand oder ein konstantes Abstandsverhältnis zu dem Objekt aufweisen. Unter Berücksichtigung des Abstandsverhältnisses oder der relativen Position des ersten Referenzsternes zu dem zweiten Referenzstern und/oder unter Berücksichtigung der Form und/oder Größe des Objektteils kann auf die Position des Objektteils relativ zu dem Referenzkoordinatensystem, wie dem ersten Referenzstern, geschlossen werden, so dass das Objektteil oder Knochenstrukturteil bezüglich des Patientendatensatzes registriert ist und Positionsveränderungen oder Verschiebungen des Objektteils relativ zu dem Objekt in dem Patientendatensatz dargestellt werden können.

Wird das Objektteil oder das Knochenstrukturteil von dem Objekt oder der Knochenstruktur abgelöst oder abgenommen oder entfernt und im Raum bewegt, so kann das IGS-System oder Navigationssystem oder Trackingsystem die Position oder Positionsinformationen des an dem Objektteil angeordneten zweiten Referenzsterns und somit die Position oder Positionsinformationen des Objektteils oder des Knochenstrukturteils ermitteln. Als Positionsinformation des Objektteils können zwei-und/oder dreidimensionale Bilder des Objektteils und/oder Abstandscharakteristika oder Abstände des Objektteils von Teilen des Objekts oder von Strukturen des Objekts und/oder Richtungen oder Bewegungsrichtungen des Objektteils und/oder Orientierungen des Objektteils relativ zu dem Objekt ermittelt werden und insbesondere in dem Patientendatensatz, wie den Aufnahmen des Objekts, dargestellt werden. Beispielsweise kann der Bewegungsverlauf des Objektteils zwei- oder dreidimensional in dem Patientendatensatz oder in den Patientenaufnahmen mittels des segmentierten Objektteils dargestellt werden. Auch können Entfernungen oder Abstände oder Koordinaten des Objektteils im Raum oder bezüglich des Objekts oder der Oberfläche des Objekts oder der Struktur des Objekts ermittelt und dargestellt werden.

Bei einem Verfahren zum Versetzen oder Neupositionieren oder Austauschen des Objektteils in dem Referenzobjekt an eine vorgegebene oder vorbestimmte Position, wird zum vorzugsweise genauen oder exakten Platzieren oder Versetzen des Objektteils das beschriebene Verfahren zum Tracken des Objektteils und zum Ermitteln und Darstellen oder Ausgeben von Positionsinformationen des Objektteils verwendet.

In dem ermittelten oder bereitgestellten Patientendatensatz der Region um das Objektteil oder des gesamten Objekts oder eines Teils des Objekts wird das in dem Patientendatensatz markierte Objektteil, zum Beispiel mittels einer Planungssoftware, an die vorgegebene oder vorbestimmte oder ausgewählte Position versetzt, so dass das markierte oder segmentierte Objektteil in dem Patientendatensatz des Objekts gesetzt oder neu positioniert oder umpositioniert wird und zum Beispiel farbig hervorgehoben wird.

Wird das Objektteil oder das Knochenstrukturteil von dem Objekt oder der Knochenstruktur gelöst oder entfernt und zum Beispiel verändert oder mit einem neuen Objektteil ausgetauscht, kann es basierend auf den mittels des IGS-Systems oder des Navigationssystems ermittelten Positionsinformationen des Objektteils an die vorgegebene oder vorbestimmte Position versetzt oder platziert werden. Beispielsweise kann das ausgetauschte oder veränderte Objektteil oder das unveränderte Objektteil in dem Patientendatensatz dargestellt werden und solange bewegt werden, bis es in dem Patientendatensatz und in dem realen Objekt die vorgegebene oder vorbestimmte Position erreicht hat. Zur genauen Auffinden der vorgegebenen oder vorbestimmten Position in dem realen Objekt dient vorzugsweise die Darstellung des Objektteils und des Objekts in dem Patientendatensatz. Beispielsweise können auch Richtungen oder Orientierungen angegeben oder dargestellt werden, welche angeben, wie das Objektteil weiterbewegt werden muss, um an die vorgegebene oder vorbestimmte Position zu gelangen. Unter Berücksichtigung der Positionsinformationen des Objektteils kann die Positionierung oder die Neupositionierung des Objektteils an die vorgegebene oder vorbestimmte Position erfolgen.

Nach dem Befestigen oder Verbinden des Objektteils mit dem Referenzobjekt an der gewünschten oder vorgegebenen Position kann der zweite Referenzstern von dem Objektteil entfernt werden und der erste Referenzstern von dem Objekt entfernt werden.

Die erfindungsgemäße Vorrichtung zum Tracken oder Verfolgen und Versetzen mindestens eines starren Objektteils, wie eines starren Körper- oder Knochenstrukturteils eines Patienten, ist in Anspruch 1 definiert und umfasst ein bildgestütztes Navigationssystem oder IGS-System (Image-Guided-Surgery-System) und eine Anzeigerichtung, wie einen Bildschirm oder Monitor, welche auch in das Navigationssystem integriert sein kann. Das Objektteil oder Knochenstrukturteil befindet sich ablösbar oder abnehmbar an einem starren Referenzobjekt, wie einer starren Körper- oder Knochenstruktur, des Patienten. In die erfindungsgemäße Vorrichtung kann ein bekannter oder abgespeicherter Patientendatensatz, wie ein CT-Datensatz eines Patienten, beispielsweise über eine Eingabevorrichtung eingegeben werden oder die erfindungsgemäße Vorrichtung kann zum Beispiel als Eingabevorrichtung ein medizinisches Bildgebungssystem, wie einen Computertomographen (CT), umfassen mittels dessen die Vorrichtung einen Patientendatensatz, wie eine Vielzahl von Computertomographie-Aufnahmen, des Objekts oder eines Teil des Objekts oder einer Region um das Objektteil ermitteln kann. Der eingegebene oder ermittelte Patientendatensatz wird auf der Anzeigevorrichtung, welche mit dem IGS-System oder Navigationssystem drahtgebunden oder drahtlos verbunden ist oder in diese integriert ist, angezeigt oder dargestellt. In dem angezeigten oder dargestellten Patientendatensatz kann das Objektteil oder das Knochenstrukturteil markiert werden, indem zum Beispiel auf der Anzeigevorrichtung, wie einem Touchscreen, das Objektteil oder Knochenstrukturteil ausgewählt wird, oder indem der Name oder die Koordinaten oder sonstige das Objektteil oder das Knochenstrukturteil bezeichnende Informationen eingegeben oder ausgewählt werden.

Ist ein erster Referenzstern auf dem Referenzobjekt in einem festen Abstandsverhältnis zu dem Objektteil angeordnet, können aus durch Berührung oder berührungslos erfassten Referenzpunkten auf dem Referenzobjekt mittels des IGS-Systems oder des bildgestützten Navigationssystems dreidimensionale Raumpositionen der Referenzpunkte des Referenzobjekts ermittelt werden. Die dreidimensionalen Raumpositionen der Referenzpunkte können bezüglich eines Referenz- oder Weltkoordinatensystems definiert werden, dessen Ursprung zum Beispiel in dem ersten Referenzstern liegt. Sind die dreidimensionalen Raumpositionen der Referenzpunkte des Referenzobjekts dem Navigationssystem bekannt, können die Raumpositionen den korrespondierenden Punkten oder Stellen in dem Patientendatensatz zugeordnet werden oder das Referenzobjekt kann mit dem in der Anzeigevorrichtung angezeigten Patientendatensatz registriert werden, so dass Veränderungen oder Verschiebungen des Objekts oder in dem Objekt oder Bewegungen relativ zu dem Objekt in dem Patientendatensatz auf der Anzeigevorrichtung angezeigt werden können.

Ist an dem Objektteil ein zweiter Referenzstern angeordnet, so kann das IGS-System oder bildgestütztes Navigationssystem die Position des zweiten Referenzsterns relativ zu dem ersten Referenzstern ermitteln, so dass die Position des zweiten Referenzsterns oder des Objektteils relativ zu einem Referenz- oder Weltkoordinatensystem von dem IGS-System oder bildgestützten Navigationssystem ermittelt werden kann. Beispielsweise kann das Navigationssystem auch basierend auf der ermittelten Position des zweiten Referenzsterns in dem Referenzkoordinatensystem und auf der Form und/oder Größe des Objektteils oder Knochenstrukturteils auf die Lage des Objektteils im Raum relativ zu dem Referenzkoordinatensystem schließen, so dass Objektteil bezüglich des Referenzkoordinatensystem registriert werden oder sein kann. Das Objektteil kann ohne Erfassung von Punkten auf dem Objektteil bezüglich des Patientendatensatzes registriert werden, so dass Bewegungen oder Verschiebungen des Objektteils relativ zu dem Objekt auf der Anzeigevorrichtung anhand des Patientendatensatzes dargestellt werden können.

Wird das Objektteil oder Knochenstrukturteil von dem Objekt abgenommen oder entfernt, kann das Navigationssystem oder IGS-System die Position oder Bewegung des Objektteils im Raum ermitteln oder verfolgen und kann die Position oder Bewegung oder Positionsinformation des Objektteils in dem Patientendatensatz auf der Anzeigevorrichtung darstellen. Beispielsweise können auf der Anzeigevorrichtung zwei- oder dreidimensionale Bilder des Bewegungsablaufs oder der Bewegung des Objektteils dargestellt werden oder es können Koordinaten oder Richtungen angegeben werden, in welche das Objektteil bewegt wird oder werden soll, um beispielsweise eine genaue Positionierung des Objektteils an der vorgegebenen oder vorbestimmten Wunschposition zu gewährleisten. Auch kann das Objektteil bewegt werden und der Bewegungsablauf kann auf der Anzeigevorrichtung verfolgt werden. Weiter kann zum Beispiel durch einen medizinischen Eingriff das Objektteil verändert oder ausgetauscht werden und so in dem Patientendatensatz auf der Anzeigevorrichtung verfolgt werden, dass das Objektteil an der ursprünglichen Position oder an einer vorgegebenen oder gewünschten neuen Position eingesetzt werden kann.

Auch kann die erfindungsgemäße Vorrichtung ein Positionierungssystem umfassen, welches den ersten und zweiten Referenzstern zum Beispiel automatisch an dem Objekt bzw. Objektteil anbringen oder anordnen kann und/oder davon entfernen kann.

Die Erfindung wird nachfolgend anhand eines bevorzugten Ausführungsbeispiels beschrieben werden. Es zeigen:

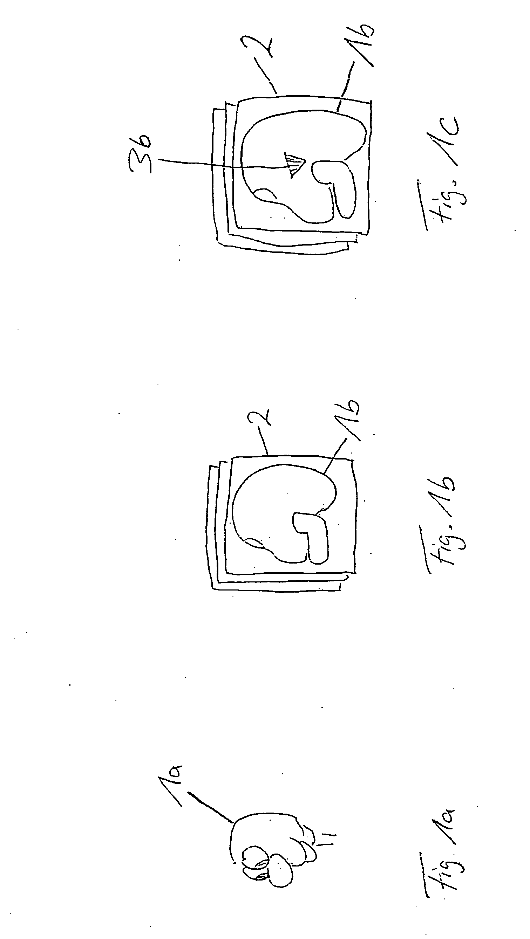

- Figuren 1a bis 1g: den Ablauf eines Verfahrens zum Verfolgen eines Schläfenbeins eines Patienten;

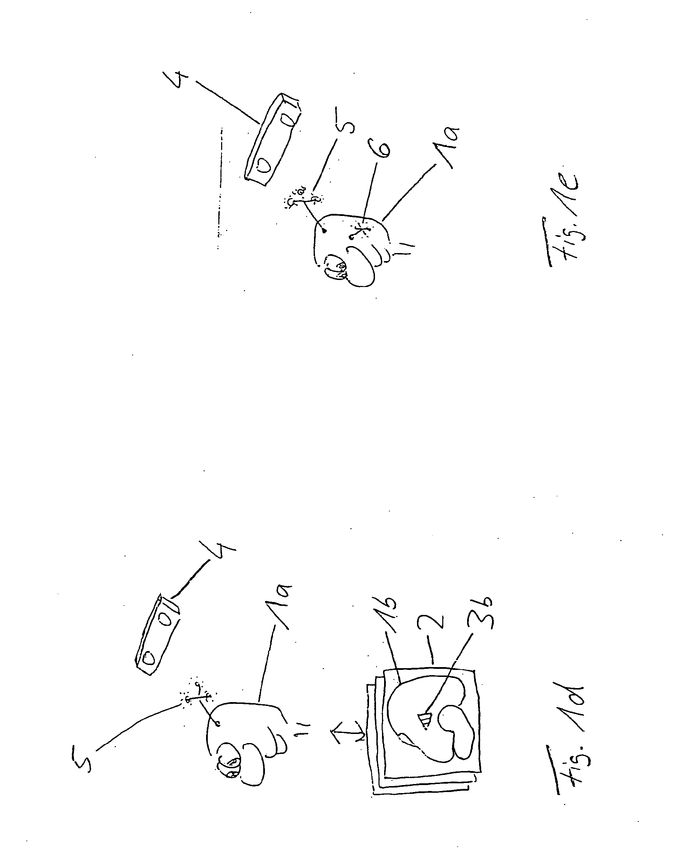

Die Figuren 1a bis 1g zeigen einen Ablauf eines Verfahrens. In Figur 1a ist der Kopf 1a eines Patienten dargestellt, von dem Computertomographie-Aufnahmen (CT-Aufnahmen), gemacht werden oder gemacht wurden, um den Schädelknochen 1b des Patienten in einem in Figur 1b gezeigten Patientendatensatz 2 aufzunehmen. In dem Patientendatensatz, wie einer Vielzahl von CT-Bildern 2, wird wie in Figur lc gezeigt, der zu verfolgende oder zu ersetzende Knochen oder das Schläfenbein 3b definiert und markiert und zum Beispiel auch von dem Patientendatensatz 2 segmentiert. Wird an dem Kopf 1a des Patienten, wie in Figur 1d dargestellt, ein erster Referenzstern 5 angebracht, kann die Position des ersten Referenzsterns 5 von einer Kamera oder einem Navigationssystem 4 erfasst werden und die dreidimensionale Raumposition des ersten Referenzsterns 5 kann bezüglich eines Referenzkoordinatensystems, wie dem Ursprung des ersten Referenzsterns 5, definiert werden. Durch Erfassen von Referenzpunkten auf dem Kopf 1a des Patienten können die Referenzpunkte den korrespondierenden Punkten in den CT-Bildern 2 zugeordnet werden, so dass der Kopf 1a oder Schädelknochen 1b des Patienten bezüglich der CT-Bilder 2 des Schädelknochens 1b registriert werden.

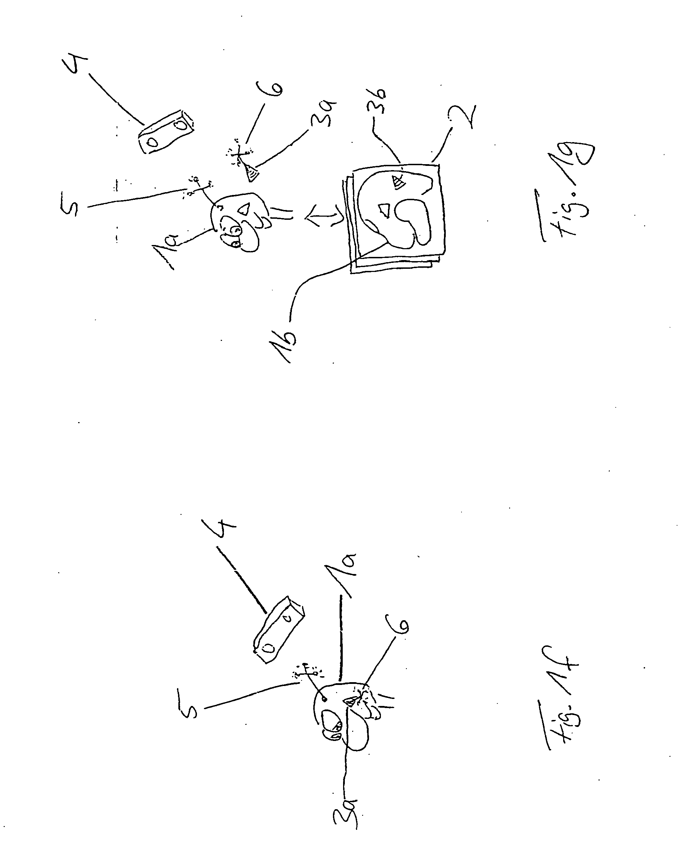

Wird an dem Schläfenbein 3a, wie in Figur 1e gezeigt, ein zweiter Referenzstern 6 angeordnet, so kann die Position des zweiten Referenzsterns 6 und die Position des Schläfenbeins 3a im Raum von dem Navigationssystem 4 ermittelt werden, siehe Figur 1e und 1f. Wird das Schläfenbein 3a von dem Schädelknochen 1b entfernt oder abgelöst, so kann das Navigationssystem 4 die dreidimensionale Position des Schläfenbeins 3a im Raum mittels des zweiten Referenzsterns 6 ermitteln und kann den Bewegungsablauf oder die Position des Schläfenbeins 3b in den CT-Bildern 2 auf einer Anzeigevorrichtung, wie in Figur 1g gezeigt, darstellen. Somit kann ohne Erfassung von Referenzpunkten auf dem Schläfenbein 3a und ohne Zuordnung der Referenzpunkte des Schläfenbeins 3a zu korrespondierenden Punkten in dem Patientendatensatz 2 das Schädelbein 3a im Raum verfolgt werden oder die Position des Schläfenbeins 3a kann ermittelt werden und in den CT-Bildern 2 dargestellt werden. Auch kann zum Beispiel durch einen medizinischen Eingriff das Schläfenbein 3a verändert oder ausgetauscht werden oder es kann ein Ersatz, wie ein Implantat, des Schläfenbeins 3a hergestellt werden und an der markierten Ausgangsposition oder an einer im Patientendatensatz 2 markierten neuen vorgegebenen oder gewünschten Position eingesetzt werden.

## Patentansprüche

1. Vorrichtung zum Tracken und Versetzen mindestens eines starren Objektteils (3a), insbesondere eines starren Körper- oder Knochenstrukturteils eines Patienten, welches von einem starren Referenzobjekt (1a), insbesondere einer starren Körper- oder Knochenstruktur des Patienten, ablösbar oder abnehmbar ist, mit:
einem bildgestützten Navigationssystem (4) oder einem Image Guided Surgery (ins)-System, zum
- Erfassen von Referenzpunkten auf dem Referenzobjekt (1a), auf welchem ein erster Referenzstern (5) in einer relativ zu dem Objektteil (3a) feststehenden Position, welche sich auf dem Referenzobjekt (1a) befindet, angeordnet ist;
- Ermitteln der dreidimensionalen Raumpositionen der Referenzpunkten des Referenzobjekts (1a) aus den erfassten Referenzpunkten des Referenzobjekts (1a);
- Zuordnen der dreidimensionalen Raumpositionen der Referenzpunkte des Referenzobjekts (1a) zu den korrespondierenden Punkten in dem Patientendatensatz (2); und
- Ermitteln von Positionsinformationen des Objektteils (3a) oder Tracken des Objektteils (3a) basierend auf den Positionsinformationen eines an dem Objektteil (3a)
angeordneten zweiten Referenzsterns (6), und unter Berücksichtigung des Abstandsverhältnisses oder der relativen Position des ersten Referenzsterns (5) zu dem zweiten Referenzstern (6) und/oder unter Berücksichtung der Form und/oder Größe des Objektteils (3a), so dass das Objektteil (3a) bezüglich des Patientendatensatzes (2) registriert ist ; und
einer Anzeigevorrichtung, welche mit dem bildgestützten Navigationssystem (4) oder IGS-System verbunden ist, zum Anzeigen des Patientendatensatzes (2) und der Positionsinformationen des Objektteils (3a).

2. Vorrichtung nach dem vorhergehenden Anspruch mit einer Eingabevorrichtung,
welche mit dem bildgestützten Navigationssystem (4) oder IGS-System verbunden ist, zum Eingeben des Patientendatensatzes (2).

3. Vorrichtung nach dem vorhergehenden Anspruch, wobei die Eingabevorrichtung als medizinisches Bildgebungssystem, insbesondere als ein CT, ausgebildet ist, mittels welchem der Patientendatensatz (2) ermittelt werden kann.

4. Vorrichtung nach einem der drei vorhergehenden Ansprüche mit einem Positionierungssystem zum Anbringen oder Anordnen des ersten Referenzsterns (5) an dem Referenzobjekt (1a) und zum Anbringen oder Anordnen des zweiten Referenzsterns (6) an dem Objektteil (3a).

## Claims

1. A device for tracking and offsetting at least one rigid object part (3a), in particular a rigid body or bone structure part of a patient, which can be detached or removed from a rigid reference object (1a), in particular a rigid body or bone structure of the patient, comprising an image-assisted navigation system (4) or an image-guided surgery (IGS) system for:
- detecting reference points on the reference object (1a), on which a first reference star (5) is arranged in a position which is fixed relative to the object part (3a) and situated on the reference object (1a);
- ascertaining the three-dimensional spatial positions of the reference points of the reference object (1a) from the detected reference points of the reference object (1a);
- assigning the three-dimensional spatial positions of the reference points of the reference object (1a) to the corresponding points in the patient data set (2); and
- ascertaining positional information of the object part (3a) or tracking the object part (3a), based on the positional information of a second reference star (6) arranged on the object part (3a) and by taking into account the distance relationship or the position of the first reference star (5) relative to the second reference star (6) and/or by taking into account the shape and/or size of the object part (3a), such that the object part (3a) is registered with respect to the patient data set (2); and
comprising a display device, which is connected to the image-assisted navigation system (4) or IGS system, for displaying the patient data set (2) and the positional information of the object part (3a).

2. The device according to the preceding claim, comprising an input device which is connected to the image-assisted navigation system (4) or IGS system, for inputting the patient data set (2).

3. The device according to the preceding claim, wherein the input device is formed as a medical imaging system, in particular as a CT, by means of which the patient data set (2) can be ascertained.

4. The device according to any one of the preceding three claims, comprising a positioning system for attaching the first reference star (5) to the reference object (1a) or arranging the first reference star (5) on the reference object (1a) and for attaching the second reference star (6) to the object part (3a) or arranging the second reference star (6) on the object part (3a).

## Revendications

1. Dispositif pour suivre et déplacer au moins une partie d'objet (3a) rigide, en particulier une partie corporelle ou partie de structure osseuse rigide d'un patient, qui peut être détachée ou enlevée d'un objet de référence rigide (1a), en particulier d'une structure corporelle ou structure osseuse rigide du patient, avec :
un système de navigation (4) assisté par imagerie ou avec un système Image Guided Surgery (IGS), pour
- relever des points de référence sur l'objet de référence (1a) sur lequel est disposée une première étoile de référence (5) dans une position fixe par rapport à la partie d'objet (3a) qui se trouve sur l'objet de référence (1a) ;
- déterminer les positions spatiales tridimensionnelles des points de référence de l'objet de référence (1a) à partir des points de référence relevés de l'objet de référence (1a) ;
- affecter les positions spatiales tridimensionnelles des points de référence de l'objet de référence (1a) aux points correspondants dans l'ensemble des données (2) du patient ; et
- déterminer des informations de position de la partie d'objet (3a) ou suivre la partie d'objet (3a) sur la base des informations de position d'une seconde étoile de référence (6) disposée sur la partie d'objet (3a), et, en tenant compte du rapport de distance ou de la position relative de la première étoile de référence (5) par rapport à la seconde étoile de référence (6) et/ou en tenant compte de la forme et/ou de la taille de la partie d'objet (3), de manière que la partie d'objet (3) soit référencée par rapport à l'ensemble de données (2) du patient ; et
avec un dispositif d'affichage qui est relié au système de navigation (4) assisté par imagerie ou système IGS, pour afficher l'ensemble de données (2) du patient et les informations de position de la partie d'objet (3a).

2. Dispositif selon la revendication 1 précédente avec un dispositif de saisie qui est relié au système de navigation (4) assisté par imagerie ou système IGS, pour introduire l'ensemble de données (2) du patient.

3. Dispositif selon la revendication 2, dans lequel le dispositif de saisie est réalisé sous la forme d'un système d'imagerie médical, en particulier sous la forme d'un CT au moyen duquel il est possible de déterminer l'ensemble de données (2) du patient.

4. Dispositif selon l'une quelconque des revendications 1 à 3 avec un système de positionnement pour appliquer ou disposer la première étoile de référence (5) sur l'objet de référence (1a) et pour appliquer ou disposer la seconde étoile de référence (6) sur la partie d'objet (3a).
